Europäisches Patentamt

European Patent Office

Office européen des brevets

(19)

(11) EP 0 564 558 B1

(12) EUROPEAN PATENT SPECIFICATION

(45) Date of publication and mention
of the grant of the patent:
**17.04.1996 Bulletin 1996/16**

(21) Application number: 92903193.8

(22) Date of filing: **18.12.1991**

(51) Int Cl.6: **G01L 5/04**, G01L 5/10,
G01N 3/20, G01N 33/36,
G01N 33/34

(86) International application number:
**PCT/US91/09765**

(87) International publication number:
**WO 92/12406 (23.07.1992 Gazette 1992/19)**

(54) **FIBER ORIENTATION SENSOR**

FASERORIENTIERUNGSSENSOR

CAPTEUR D'ORIENTATION DE FIBRES

(84) Designated Contracting States:
**DE FR GB IT SE**

(30) Priority: **28.12.1990 US 635305**
**28.12.1990 US 635291**

(43) Date of publication of application:
**13.10.1993 Bulletin 1993/41**

(73) Proprietor: **MEASUREX CORPORATION**
**Cupertino, California 95014 (US)**

(72) Inventors:
- **CRESSON, Thierry, M.**
 **Cupertino, CA 95014 (US)**
- **CHASE, Lee, M.**
 **Los Gatos, CA 95030 (US)**

- **ANDERSON, Leonard, M.**
 **San Jose, CA 95130 (US)**
- **GOSS, John, D.**
 **San Jose, CA 95123 (US)**

(74) Representative: **Allman, Peter John et al**
**MARKS & CLERK,**
**Sussex House,**
**83-85 Mosley Street**
**Manchester M2 3LG (GB)**

(56) References cited:
**EP-A- 0 311 507**          **GB-A- 934 328**
**US-A- 2 834 203**          **US-A- 3 158 021**
**US-A- 3 204 454**          **US-A- 3 677 076**
**US-A- 4 581 575**          **US-A- 4 864 851**
**US-A- 4 866 984**          **US-A- 4 936 140**
**US-A- 4 936 141**          **US-A- 4 970 895**
**US-A- 4 991 432**          **US-A- 5 010 766**

**Description**

The present invention relates to a sensor for determining directional variations in a physical characteristic in a moving sheet of material. In particular, the present invention relates to a sensor for determining the orientation of fibers in a moving sheet of fibrous material based upon the directional variation of a physical characteristic, and a method of using the sensor.

Many of the qualities and characteristics of a fibrous sheet material depend, at least in part, on the orientation of the fibers forming the material. Furthermore, the orientation of the fibers within the sheet material can, to a certain degree, be controlled during the manufacturing process.

For example, paper is typically formed by ejecting a slurry of water and paper pulp fibers from a headbox onto a moving porous belt, frequently called a "wire". The water component of the slurry drips through the wire leaving a mat of paper fibers on the wire. The mat is then subject to additional processing, such as pressing, drying, calendering, coloring, coating or the like to form a finished paper product. Many of the qualities and characteristics of the finished paper product are related to the orientation of the paper pulp fibers in the original mat.

That is, as the slurry is ejected onto the moving wire the paper pulp fibers may have a tendency or probability to become longitudinally oriented in one particular direction. If this tendency is high, a large number of fibers are oriented in one particular direction and the resulting paper product will be highly anisotropic. If the probability or tendency of the fibers to be oriented in any particular direction is low, that is, the fibers are truly in a random orientation, the resulting paper product will be isotropic. The properties, such as tensile strength, of anisotropic products may exhibit directional variations while the properties of isotropic products tend to be independent of direction. Depending on the intended application, the optimum degree of anisotropism may vary.

Furthermore, for anisotropic products, the direction of predominant fiber orientation is also important. The directionality of the properties of anisotropic products usually depends on the predominant direction of fiber orientation. That is, if the predominant fiber orientation is at an angle to the machine direction, then the directionally dependant properties will typically exhibit either a maximum or a minimum at the same angle. Therefore, information relating to both the direction of the predominant fiber orientation, or the fiber orientation angle, as well as the degree of anisotropism is useful in evaluating fiber orientation in a fibrous sheet of material. Typically, in papermaking, the fiber orientation angle is defined as the angle between the machine direction, the direction of the moving wire, and the predominant direction of fiber orientation.

The tendency of the paper pulp fibers to have particular orientation depends on a large number of factors, including the geometry of the headbox, the speed of the wire, the speed of the ejected slurry, the direction of the ejection of the slurry relative to the wire, and cross-currents within the slurry. Many of these factors can be controlled. Therefore, it is useful to be able to quickly and accurately measure the degree of anisotropism and the fiber orientation angle during the manufacturing process. This allows for the manufacturing process to be adjusted to achieve the desired product qualities.

One present method of determining fiber orientation involves measuring and counting the orientation of individuals fibers within the fibrous sheet. Because this method is extremely labor intensive, it is expensive, time consuming and generally impractical for use in a manufacturing situation.

Another, presently used method of measuring the fiber orientation in a fiber sheet utilizes the relationship between tensile strength and fiber orientation. In this method, the tensile strength of a sample of the fibrous sheet is tested in several directions. A polar plot of the measured tensile strengths typically results in a shape resembling an ellipse. The ratio of the major axis of the ellipse to the minor axis of the ellipse provides an indication of the degree of anisotropism of the fibers in the sheet. Further, the angle between the major axis of the ellipse and the machine direction corresponds to the fiber orientation angle.

Although this test has been found to be relatively reliable, it is destructive to the fibrous sheet, time consuming, and cannot be conducted on-line. Given the speed of modern paper making equipment, this can result in the production of large quantities of substandard product before the test results are available. Furthermore, because the fiber orientation can vary substantially in the cross direction, i.e., across the width of the wire normal to the machine direction, the test must be conducted on samples taken at various intervals, or slices, across the sheet. This further increases the time and expense of this method of determining fiber orientation.

In addition, a number of devices employing optical, sonic, X-ray, or microwave attenuation techniques have been devised to measure fiber orientation. However, such devices are typically expensive, and are not well suited for the harsh environment often found in paper mills.

EP-A-0 311 507 discloses a system and process for continuously determining the strength of paper sheet material during manufacture. A plurality of sensors detect proxies relating to various paper sheet properties including fiber orientation. Proxy measures are sensed for at least four of the properties during manufacture of the paper sheet and, for a selected paper making machine and paper grade, calculations are made of correlations between the proxy measures and laboratory tests of paper strength. Based upon the determined correlations, the predicted strength of the paper being produced by the paper making machine is calculated. EP-A-0 311 507 discloses several prior art techniques for measuring fiber orientation, including (1) the use of a laser light beam and a two-dimensional photo-detector to detect the light scattering differential between randomly oriented fibers and fibers oriented parallel to one another; (2) the use of two microwave moisture sensors with one oriented in the machine direction and the other in the cross direction; (3) detecting polarized light at right angles to the plane of the paper, reflecting the light in two planes at right angles to each other, and forming two quantities based upon the reflected light to provide an index value for the anisotropy of fiber orientation; and (4) the use of X-ray diffraction and zero-span tensile testing. EP-A-0 311 507 also discloses a four segment ring sensor with associated load cells for the purpose of detecting stress applied to the sheet to provide a "process proxy measure" for the strength of the sheet. The disclosed sensor provides strength (elastic moduli) measurements in the cross- and machine-directions. The subject publication does not suggest using a rotatable ring sensor for the determination of fiber orientation.

US-A-4 866 984 describes a sensor for continuous sensing of a quantity of paper related to its elastic modulus and stiffness including a paper support in the form of a segmented ring, and a free running wheel which depresses the paper in the center of the ring as the paper passes over it. Force transducers mounted to sense force exerted on the segments provides a measure of a characteristic of the paper related to its elastic modulus and bending stiffness. The segments are aligned so that separate determinations of machine direction and cross-direction characteristics are made. The outputs of this sensor, together with basis weight, thickness and paper velocity are used to determine the strength of the paper on a continuous basis. Separate values of machine direction and cross-direction strength can be determined, as can a burst pressure strength. In the described embodiment, the ring includes four segments, however, it is explicitly noted that a different number of segments may be provided.

Accordingly, it is an object of the present invention to provide a sensor which can provide information about the directional variation of a physical characteristic of a moving sheet of material.

Another object of the invention is to provide a sensor that is reliable and well suited for use on-line in a manufacturing environment.

Yet another object of the invention is to provide a sensor that is relatively inexpensive and easy to install, use, and maintain.

According to a first aspect of the present invention there is provided a sensor for determining information relating to directional variations in a physical characteristic of a moving sheet of material, the sensor comprising a support for supporting the moving sheet, said support defining an open region; a deflector for deflecting the sheet into the open region; and a plurality of detectors associated with the support, said detectors arranged to detect a physical parameter related to the force exerted on the support by the deflected sheet, characterized by means for rotating the support to change the orientation of the detectors to detect the physical parameter in a plurality of directions and produce signals indicative of the detected physical parameter in said plurality of directions; and electronic circuits operatively coupled to the detectors to receive said signals, wherein the circuits are adapted to determine information relating to directional variations in the physical characteristic of the sheet based upon said signals.

According to a second aspect of the present invention there is provided a method for determining information relating to directional variations in a physical characteristic of a moving sheet of material, comprising the steps of supporting one side of the moving sheet at a plurality of regions defining an unsupported region therebetween; deflecting the sheet into the unsupported region; and detecting a physical parameter related to the force of the sheet at at least two of said plurality of regions in a direction extending between said two regions; characterized by rotating the plurality of regions to change the orientation of said at least two regions; detecting the physical parameter in said direction extending between the rotated two regions; and determining the directional variation of the physical characteristic based upon the detected physical parameter in the first and second mentioned directions.

A specific embodiment of the present invention will now be described with reference to the accompanying drawings in which:

Figure 1 illustrates a scanner having a preferred embodiment of the present sensor mounted thereto for scanning the sensor repeatedly across the width of the sheet of moving material;
Figure 2 is a partially cut away side view of the sensor shown in Figure 1;
Figure 3 is a top view of the ring portion of the sensor shown in Figure 1;
Figure 4 is a cross sectional view of the ring portion taken along line 4-4 in Figure 3, and
Figure 5 is a polar plot of the extensional stiffness as determined by the sensor.

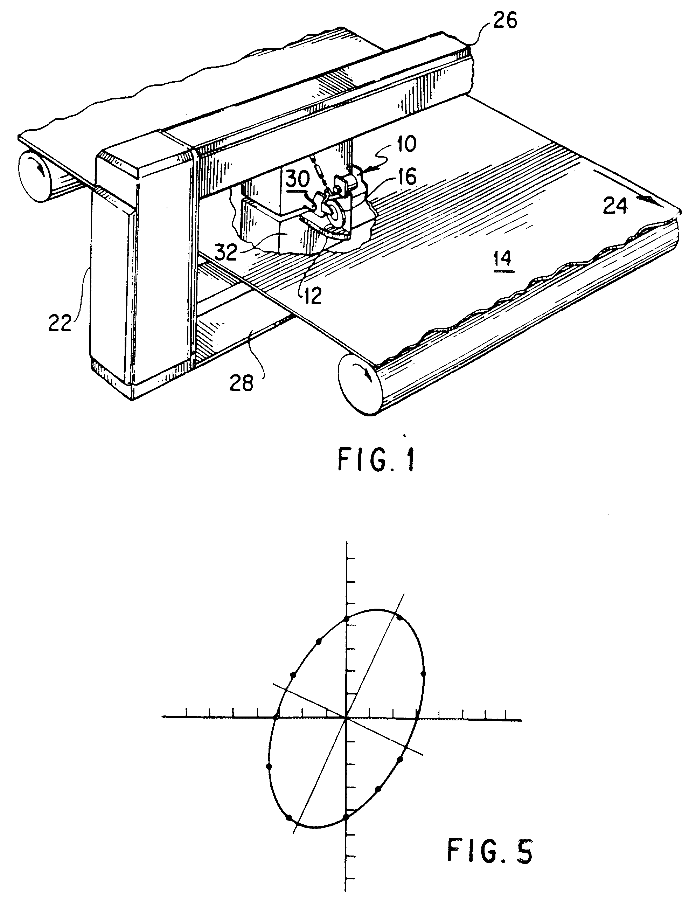

A sensor in accordance with a preferred embodiment of the present invention is indicated as reference numeral 10 in Figure 1. The illustrated sensor 10 has a sheet support ring 12 positioned on one side of a moving sheet of material 14, paper in the illustrated embodiment. A rotating wheel 16 is positioned on the other side of the moving sheet 14 to deflect the sheet into the opening defined by the ring 12. Two segments 18a and 18b are located opposite one another on the inner periphery of the ring 12. Associated with each segment 18a and 18b is a load cell 20a and 20b, respectively, which can be used to measure the force exerted by the deflected sheet 14 on each segment 18a and 18b. In this manner, the sensor 10 can detect the force exerted by the sheet 14 in the direction of the line defined by the two segments 18a and 18b.

The ring 12 is mounted on a rotatable support 19 to allow the ring to be rotated to orient the two segments 18a and 18b in any desired direction. The rotatable support 19, is not described in detail and can have a number of configurations apparent to those skilled in the art. For example, a motor and gear assembly can be used to rotate the support 19 and slip rings can facilitate any necessary electrical connections.

In the illustrated embodiment, the sensor 10 is carried by a scanning station 22 which scans the sensor 10 back and forth across the width of the sheet 14 which is moving in the direction of arrow 24. In this manner, the sepsor 10 can take readings at predetermined intervals, or slices, across the width of the sheet 14 and detect any variations in the measured physical characteristic in the cross direction.

The scanning station 22 can be one of many types well known to those skilled in the art. The illustrated scanning station 22 includes two beams 26 and 28 which extend tranversely across the width of the moving sheet 14. One beam 26 is positioned above the moving sheet 14 and the other beam 28 is positioned below the moving sheet. Upper and lower gauge support members are mounted to the upper and lower beams 26 and 28, respectively. In the illustrated embodiment, the upper gauge support member 30 carries the wheel 16 and the lower gauge support member 32 carries the ring 12. The two gauge support members 30 and 32 are positioned in vertical alignment with respect to each other to position the wheel 16 within the opening defined by the ring 12. In Figure 1, a portion of the sheet is cut away showing the relationship between the upper and lower gauge support members 30 and 32, respectively. A motor (not shown) within the scanning system drives the gauge support members 30 and 32 back and forth across the moving sheet 14 in a continuous scanning motion. The movement of the upper and lower gauge support members 30 and 32 is synchronized to maintain a constant vertical alignment between the wheel 16 and the opening defined by the ring 12 at all times during the scanning motion.

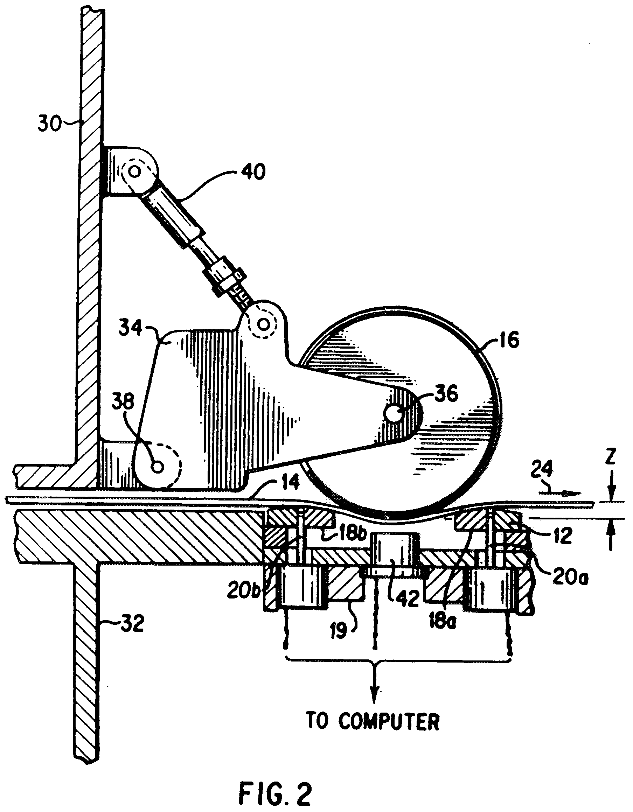

As best seen in Figure 2, the wheel 16 is mounted to wheel carrier 34 for rotation about a central axle 36. The wheel carrier 34 is pivotally mounted to the upper gauge support member 30 by pivot pin 38. An adjustable air cylinder 40 with one end fastened to the upper support member 30 and the other end fastened to the wheel carrier can be extended or retracted to vary the distance, z, which the wheel 16 deflects the moving sheet 14. Preferably, a computer (not shown) is operatively coupled to the air cylinder 40 to control the deflection distance. Preferably, the periphery of the wheel 16 is spherically convex rather than cylindrical, such that the sheet is deflected uniformly in all directions and the deflection is generally symmetrical in all directions about the lowest point of the rotating wheel 16.

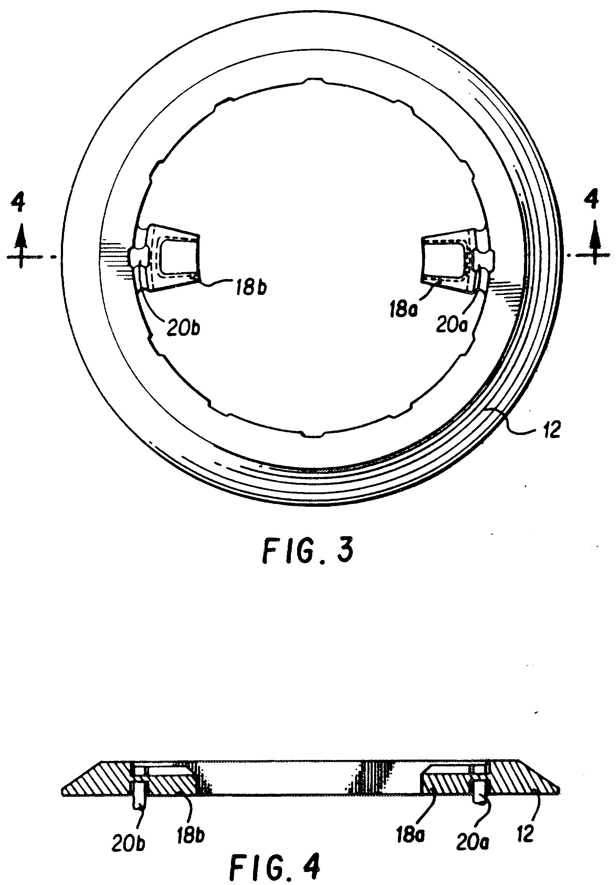

The ring 12 is carried by the lower gauge support member 32 on the opposite side of the moving sheet 14. As previously mentioned, the two support members are vertically aligned to maintain the lowest point of rotating wheel 16 substantially centered in the open region defined by the ring 12. The upper surface of the ring 12 provides a support surface for the moving sheet 14. As seen in Figure 3, two independently movable segments 18a and 18b are positioned on opposite sides of the ring 12.

When the sheet 14 is deflected, best seen in Figure 2, it exerts a force against each segment 18a and 18b. This force is detected by load cells 20a and 20b. In the illustrated embodiment cantilever load cells are shown. However, different types of load cells may be desirable in different applications of the present sensor.

In the illustrated embodiment, the two segments 18a and 18b are directly opposite one another. As a result, the two segments 18a and 18b define a line passing through the center of the ring. In this manner, the load cells associated with the segments detect the force exerted by the deflected sheet in the direction of the line defined by the segments 18a and 18b. The support ring is fixed to a rotatable mount 19. This allows the ring to be rotated to orient the two segments, and hence the direction in which the force is detected, to any desired direction. As a result, the sensor can detect directional variations in the force exerted by the deflected sheet.

Because the force detected by each load cell 20a and 20b is actually the force exerted by the deflected sheet 14 over the entire arc defined by the associated segment, the resolution of the sensor depends on the size of each segment. Further, if more than two segments are used, it is possible to detect forces exerted in more than one direction at any given orientation of the ring. In some applications, an increased number of segments may be desirable to reduce the number of ring orientations required. The illustrated embodiment uses two segments each covering an arc of approximately 30 degrees. This has been found suitable for the detection of fiber orientation in paper. However, in other applications it may be desirable to employ a different number of segments, segments of a different size, or both.

The force exerted by the deflected sheet against any particular segment is the result of three additive factors. The first factor is the stress in the sheet caused by the strain due to local distortion by the wheel when it deflects the sheet into the open region of the ring. This is the extensional stiffness of the sheet. The second factor is the tension in the sheet. It has been found that for all practical purposes the only tension in the sheet is that generated in the machine direction by the manufacturing machinery. The third additive factor is the bending stiffness of the sheet. Mathematically, the combined outputs of the load sensors corresponding to an opposing pair of segments can be expressed as follows:

$$(1) \qquad OUTPUT = (A)\,(E)\,(t)\,(F_1(z^n)) + (B)\,(T)\,(z) + (C)\,(\beta)\,(z)$$

Where:

OUTPUT = the sum of the force of the deflected sheet against the two opposing segments.

E = Young's modulus of the sheet in the direction of the segments.

t = the caliper of the sheet.

z = the distance which the sheet is deflected.

$F_1$ = a function of z raised to a power greater than one relating the extensional stiffness of the sheet (i.e., (E) (t)) to the stress in the sheet caused by sheet strain as the wheel pushes the sheet into the opening defined by the ring.

T = the tension in the sheet along the direction of the segments. Although the only tension is in the machine direction, each segment subtends an arc and will have an effective width in the machine direction. Hence, each segment will be somewhat affected by the machine directed tension. The effective width of each segment can be empirically determined if desired.

$\beta$ = the bending resistance of the sheet in the direction of the segments.

A, B, and C = empirically determined proportionality constants.

The equations above are subject to a variety of mathematical solutions with many of the variables being determined with existing sensors or gauges. For example, commonly assigned and copending U.S. Patent Application Serial No. 07/247,177, now U.S. Patent No. 5,029,469 issued July 9, 1991, discusses the solution of similar equations and describes sensors for determining sheet tension and extensional stiffness.

However, in the preferred embodiment of the present sensor, solution of the equations to find the fiber orientation of the sheet is greatly simplified. This is because it has been found that fiber orientation may be directly related to extensional stiffness, i.e., the first term of equation (1). In particular, as illustrated in Figure 5, a polar graph of the extensional stiffness of the moving sheet forms a shape which may be approximated as an ellipse. The angle between the major axis of the ellipse and the machine direction corresponds with the fiber orientation angle. Moreover, the ratio of the major axis of the ellipse to the minor axis of the ellipse is an indication of the degree of anisotropism of the sheet. As a result, by determining the extensional stiffness of the sheet in a number of directions, the present sensor can be used to determine the fiber orientation of the sheet.

The first term of equation (1) is a function of z raised to a power greater than one. However, the second and third terms, tension and bending stiffness, respectively, are linear functions of z. Therefore, if both sides of equation (1) are divided by z the second and third terms of equation become independent of z, as shown in equation (2).

$$(2) \qquad OUTPUT/z = (A)(E)(t)(F(z^{n-1})) + (B)(T) + (C) + (\beta)$$

Furthermore, two equations, (3) and (4), can be established by making two measurements with two different z values.

$$(3) \qquad OUTPUT_1/z_1 = (A)\,(E)\,(t)\,(F(z_1^{n-1})) + (B)\,(T) + (C)(\beta)$$

$$(4) \qquad OUTPUT_2/z_2 = (A)\,(E)\,(t)\,(F(z_2^{n-1})) + (B)\,(T) + (C)\,(\beta)$$

Subtracting equation (4) from equation (3) yields equation (5) and eliminates the tension and bending stiffness terms completely.

$$(5) \qquad OUTPUT_1/z_1 - OUTPUT_2/z_2 = (A)\,(E)\,(t)\,(F(z_1^{n-1})) - (F(z_2^{n-1}))$$

The right hand side of equation (5) is proportional to the extensional stiffness. It is unnecessary to evaluate the proportionality constant because it will not affect the orientation or the ratio of the major axis to the minor axis of the ellipse formed by the polar plot of the extensional stiffness. For the same reason, it is not necessary to evaluate the caliper of the sheet, t. However, the caliper can be easily and routinely determined using a number of readily available caliper gauges.

To implement the equations outlined above in connection with the preferred embodiment, the sensor 10 must make two different measurements at any given ring orientation, one at a first deflection distance, or z value, and another at a second deflection distance, different from the first. This is accomplished by adjusting the air cylinder 40 to cause the wheel to deflect the sheet a first distance into the open region defined by the ring; scanning the sensor across the width of the sheet and stopping at predetermined slices; rotating the ring to a number of desired orientations; determining the output of each load cell at each orientation at each slice; storing the outputs of each load cell at each orientation of each slice; adjusting the air cylinder to cause the wheel to deflect the sheet a second distance into the ring; scanning the sensor back across the sheet and determining the output of each load cell at each orientation at each slice; and using the stored first output, the first deflection distance, the second output, and the second deflection distance to solve equation (5) for each opposing pair of segments at each orientation and slice across the sheet.

Of course, using this method, because the sheet is moving, the two measurements at different deflections do not correspond to exactly the same part of the moving sheet. However, it has been found that under normal operating conditions, fiber orientation typically changes very slowly in the machine direction. Therefore any error introduced by taking the two different measurements at different locations along the machine direction of the sheet is negligible.

The distance which the sheet is deflected at any given adjustment may be determined by means of a displacement sensor 42. The displacement sensor can be any one of a variety of known sensors, such as an eddy current device, which uses magnetic fields to determine the position of the wheel 16 and hence the deflection distance of the sheet 14. The actual deflection distance is not critical to the invention and will vary depending on the geometry of the sensor. However, in the illustrated embodiments having a wheel with a diameter of about 12,7 cm (5 inches) and a ring defining an open region with a diameter of about 8,8 cm (3.5 inches), deflections of about 3,5 mm (0.14 inches) and about 2 mm (0.08 inches) have proved satisfactory.

In the preferred embodiment, control of the air cylinder 40, and hence the displacement of the sheet, as well as the control of the scanning station to implement the steps described above can be facilitated by an appropriately programmed computer. The computer can also be programmed to monitor the outputs from the load cells, solve the equations described above, and determine the fiber orientation of the sheet.

This detailed description is set forth only for purposes of illustrating examples of the present invention and should not be considered to limit the scope thereof in any way. Although the described embodiment has the wheel positioned above the sheet and the ring positioned below the sheet, it is equally possible, and in some circumstances may be desirable to position the ring above the sheet and the wheel below the sheet. Clearly numerous other additions, substitutions, and other modifications can be made to the invention without departing from the scope of the invention, which is defined in the appended claims.

## Claims

1. A sensor (10) for determining informtion relating to directional variations in a physical characteristic of a moving sheet (14) of material, the sensor (10) comprising a support (12) for supporting the moving sheet (14), said support (12) defining an open region; a deflector (16, 40) for deflecting the sheet (14) into the open region; and a plurality of detectors (20) associated with the support, said detectors (20) arranged to detect a physical parameter related to the force exerted on the support (12) by the deflected sheet, <u>characterized by</u> means (19) for rotating the support (12) to change the orientation of the detectors (20) to detect the physical parameter in a plurality of directions and produce signals indicative of the detected physical parameter in said plurality of directions; and electronic circuits operatively coupled to the detectors to receive said signals, wherein the circuits are adapted to determine information relating to directional variations in the physical characteristic of the sheet based upon said signals.

2. A sensor according to claim 1, wherein the deflector includes a rotatable wheel (16) disposed to deflect the sheet (14) into the open region.

3. A sensor according to claim 1 or 2 further comprising means (42) for measuring the distance which the sheet is deflected into the open region.

4. A sensor according to claim 1 or 2 further comprising means (40) for controlling the distance which the sheet is deflected into the open region.

5. A sensor according to any preceding claim, wherein the rotating means (19) orients the detectors in more than three directions to produce a signal indicative of the physical parameter in more than three directions, and wherein the electronic circuits determine the directional variations in the physical parameter based upon the more than three signals.

6. A sensor according to any preceding claim, wherein the sheet (14) is a fibrous material and the electronic circuits determine fiber orientation angles based upon the directional variations in the physical characteristic.

7. A sensor according to any preceding claim, wherein the support (12) includes at least one pair of supporting segments (18a, 18b), each supporting segment being independently movable in the direction of sheet deflection, the segments of said at least one pair of supporting segments being located opposite one another about said open region substantially along a line passing through the deflector (16) and further wherein a detector (20a, 20b) is associated with each supporting segment for detecting the physical parameter related to the force exerted by the deflected sheet in a direction along said line and producing a signal indicative of the physical parameter in said direction.

8. A sensor according to claim 7 wherein the moving sheet (14) is a fibrous material and the circuit means is adapted to determine information relating to the orientation of fibers within the moving fibrous sheet based upon the signal.

9. A sensor according to any preceding claim, wherein the support (12) and the deflector (16, 40) are adapted to be positioned on opposite sides of the moving sheet (14).

10. A sensor according to claim 9 wherein the deflector (16, 40) is adapted to deflect the sheet (14) a predetermined distance into the open region.

11. A method for determining information relating to directional variations in a physical characteristic of a moving sheet (14) of material, comprising the steps of supporting one side of the moving sheet at a plurality of regions defining an unsupported region therebetween; deflecting the sheet into the unsupported region; and detecting a physical parameter related to the force of the sheet at at least two (18a, 18b) of said plurality of regions in a direction extending between said two regions; characterized by rotating the plurality of regions to change the orientation of said at least two regions; detecting the physical parameter in said direction extending between the rotated two regions; and determining the directional variation of the physical characteristic based upon the detected physical parameter in the first and second mentioned directions.

12. A method according to claim 11 wherein the sheet is a fibrous sheet and further comprising the step of determining the fiber orientation angle based upon the directional variation of the physical characteristic.

13. A method according to claim 11 wherein the sheet is a fibrous sheet and further comprising the step of determining the degree of anisotropism of the fibers in the sheet based upon the directional variation of the physical characteristic.

**Patentansprüche**

1. Sensor (10) zum Bestimmen Von Informationen, die Richtungsschwankungen eines physikalischen Kennzeichens eines sich bewegenden Bogens (14) eines Materials betreffen, wobei der Sensor (10) folgendes umfaßt: eine Stütze (12) zum Stützen des sich bewegenden Bogens (14), wobei die Stütze (12) einen offenen Bereich definiert; eine Ablenkvorrichtung (16, 40) zum Ablenken des Bogens (14) in den offenen Bereich; und eine Mehrzahl von Detektoren (20), die der Stütze zugeordnet sind, wobei die Detektoren (20) dazu angeordnet sind, einen physikalischen Parameter zu erfassen, der mit der Kraft in Beziehung steht, die durch den abgelenkten Bogen auf die Stütze (12) ausgeübt wird, gekennzeichnet durch ein Mittel (19) zum Drehen der Stütze (12), um die Ausrichtung der Detektoren (20) zu ändern, um den physikalischen Parameter in einer Mehrzahl von Richtungen zu erfassen und Signale zu produzieren, die den erfaßten physikalischen Parameter in dieser Mehrzahl von Richtunqen anzeigen, und elektronische Schaltkreise, die in Wirkverbindung mit den Detektoren stehen, um die Signale zu empfangen, wobei die Schaltkreise dafür ausgelegt sind, auf der Grundlage der Signale Informationen zu bestimmen, die Richtungsschwankungen des physikalischen Kennzeichens des Bogens betreffen.

2. Sensor nach Anspruch 1, bei dem die Ablenkvorrichtung ein drehbares Rad (16) umfaßt, das dazu angeordnet ist, den Bogen (14) in den offenen Bereich abzulenken.

3. Sensor nach Anspruch 1 oder 2, der ferner ein Mittel (42) zum Messen des Abstands umfaßt, um den der Bogen in den offenen Bereich abgelenkt wird.

4. Sensor nach Anspruch 1 oder 2, der ferner ein Mittel (40) zum Steuern des Abstands umfaßt, um den der Bogen in den offenen Bereich abgelenkt wird.

5. Sensor nach einem der vorhergehenden Ansprüche, bei dem das Drehmittel (19) die Detektoren in mehr als drei Richtungen ausrichtet, um ein Signal zu produzieren, das den physikalischen Parameter in mehr als drei Richtungen anzeigt, und bei dem die elektronischen Schaltkreise die Richtungsschwankungen des physikalischen Parameters auf der Grundlage der mehr als drei Signale bestimmen.

6. Sensor nach einem der vorhergehenden Ansprüche, bei dem der Bogen (14) ein faseriges Material ist und die elektronischen Schaltkreise auf der Grundlage der Richtungsschwankungen des physikalischen Kennzeichens Faserausrichtungswinkel bestimmen.

7. Sensor nach einem der vorhergehenden Ansprüche, bei dem die Stütze (12) mindestens ein Paar Stütz-Segmentteile (18a, 18b) umfaßt, wobei jedes Stütz-Segmentteil unabhängig in Richtung der Bogenablenkung beweglich ist, wobei die Segmentteile des mindestens einen Paars von Stütz-Segmentteilen um den offenen Bereich herum sich gegenüberliegen, und zwar im wesentlichen entlang einer Geraden, die durch die Ablenkvorrichtung (16) geht, und bei dem ferner jeder Stütz-Segmentteil ein Detektor (20a, 20b) zugeordnet ist zum Erfassen des physikalischen Parameters, der mit der Kraft in Beziehung steht, die vom abgelenkten Bogen in einer Richtung entlang der Geraden ausgeübt wird, und zum Produzieren eines Signals, das den physikalischen Parameter in dieser Richtung anzeigt.

8. Sensor nach Anspruch 7, bei dem der sich bewegende Bogen (14) ein faseriges Material ist und das Schaltkreismittel dafür ausgelegt ist, auf der Grundlage des Signals Informationen zu bestimmen, welche die Faserausrichtung innerhalb des sich bewegenden faserigen Bogens betreffen.

9. Sensor nach einem der vorhergehenden Ansprüche, bei dem die Stütze (12) und die Ablenkvorrichtung (16, 40) dafür ausgelegt sind, an entgegengesetzten Seiten des sich bewegenden Bogens (14) positioniert zu werden.

10. Sensor nach Anspruch 9, bei dem die Ablenkvorrichtung (16, 40) dafür ausgelegt ist, den Bogen (14) um einen vorgegebenen Abstand in den offenen Bereich abzulenken.

11. Verfahren zum Bestimmen von Informationen, die Richtungsschwankungen eines physikalischen Kennzeichens eines sich bewegenden Bogens (14) eines Materials betreffen, das die folgenden Schritte umfaßt: das Stützen einer Seite des sich bewegenden Bogens in einer Mehrzahl von Bereichen, die zwischen sich einen ungestützten Bereich definieren; das Ablenken des Bogens in den ungestützten Bereich; und das Erfassen eines physikalischen Parameters, der mit der Kraft des Bogens in Beziehung steht, in mindestens zwei (18a, 18b) der Mehrzahl von Bereichen in einer Richtung, die sich zwischen den beiden Bereichen erstreckt; gekennzeichnet durch das Drehen der Mehrzahl von Bereichen, um die Ausrichtung der mindestens zwei Bereiche zu ändern; das Erfassen des physikalischen Parameters in der Richtung, die sich zwischen den beiden gedrehten Bereichen erstreckt; und das Erfassen der Richungsschwankung des physikalischen Kennzeichens auf der Grundlage des erfaßten physikalischen Parameters in der ersten und zweiten erwähnten Richtung.

12. Verfahren nach Anspruch 11, bei dem der Bogen ein faseriger Bogen ist und das ferner den Schritt umfaßt, auf der Grundlage der Richtungsschwankung des physikalischen Kennzeichens den Faserausrichtungswinkel zu bestimmen.

13. Verfahren nach Anspruch 11, bei dem der Bogen ein faseriger Bogen ist und das ferner den Schritt umfaßt, auf der Grundlage der Richtungsschwankung des physikalischen Kennzeichens den Anisotropiegrad der Fasern im Bogen zu bestimmen.

## Revendications

1. Capteur (10) servant à déterminer des informations concernant des variations directionnelles d'une caractéristique physique d'une feuille mobile (14) de matériau, le capteur (10) comprenant un support (12) servant à supporter la feuille mobile (14), ledit support (12) définissant une région ouverte ; un déflecteur (16, 40) servant à défléchir la feuille (14) dans la région ouverte ; et une pluralité de détecteurs (20) associés au support, lesdits détecteurs (20) arrangés pour détecter un paramètre physique lié à la force appliquée sur le support (12) par la feuille défléchie, caractérisé par un moyen (19) servant à faire effectuer une rotation au support (12) pour changer l'orientation des détecteurs (20) pour détecter le paramètre physique dans une pluralité de directions et produire des signaux indicatifs du paramètre physique détecté dans ladite pluralité de directions ; et des circuits électroniques accouplés activement aux détecteurs pour recevoir lesdits signaux, dans lequel les circuits sont adaptés pour déterminer les informations concernant les variations directionnelles de la caractéristique physique de la feuille sur la base desdits signaux.

2. Capteur selon la revendication 1, dans lequel le déflecteur comporte une roue rotative (16) disposée pour défléchir la feuille (14) dans la région ouverte.

3. Capteur selon la revendication 1 ou 2, comprenant en outre un moyen (42) servant à mesurer la distance sur laquelle la feuille est défléchie dans la région ouverte.

4. Capteur selon la revendication 1 ou 2, comprenant en outre un moyen (40) servant à contrôler la distance sur laquelle la feuille est défléchie dans la région ouverte.

5. Capteur selon une revendication précédente quelconque, dans lequel le moyen rotatif (19) oriente les détecteurs dans plus de trois directions pour produire un signal indicatif du paramètre physique dans plus de trois directions, et dans lequel les circuits électroniques déterminent les variations directionnelles du paramètre physique sur la base des plus de trois signaux.

6. Capteur selon une revendication précédente quelconque, dans lequel la feuille (14) est un matériau fibreux et les circuits électroniques déterminent l'orientation des fibres, les angles se basant sur les variations directionnelles de la caractéristique physique.

7. Capteur selon une revendication précédente quelconque, dans lequel le support (12) comporte au moins une paire de segments de support (18a, 18b), chaque segment de support étant mobile indépendamment dans la direction de déflexion de la feuille, les segments de ladite au moins une paire de segments de support étant situés l'un en face de l'autre autour de ladite région ouverte sensiblement le long d'une ligne passant par le déflecteur (16) et en outre dans lequel un détecteur (20a, 20b) est associé à chacun des segments de support servant à détecter le paramètre physique lié à la force appliquée par la feuille défléchie dans une direction le long de ladite ligne et produisant un signal indicatif du paramètre physique dans ladite direction.

8. Capteur selon la revendication 7, dans lequel la feuille mobile (14) est un matériau fibreux et le moyen de circuit est adapté pour déterminer les informations concernant l'orientation des fibres dans la feuille fibreuse mobile sur la base du signal.

9. Capteur selon une revendication précédente quelconque, dans lequel le support (12) et le déflecteur (16, 40) sont adaptés pour être positionnés sur des côtés opposés de la feuille mobile (14).

10. Capteur selon la revendication 9, dans lequel le déflecteur (16, 40) est adapté pour défléchir la feuille (14) sur une distance prédéterminée dans la région ouverte.

11. Procédé servant à déterminer les informations concernant les variations directionnelles d'une caractéristique physique d'une feuille mobile (14) de matériau, comprenant les étapes consistant à supporter un côté de la feuille mobile au niveau d'une pluralité de régions définissant une région non supportée entre elles ; à défléchir la feuille dans la région non supportée ; et à détecter un paramètre physique lié à la force de la feuille au niveau d'au moins deux (18a, 18b) parmi ladite pluralité de régions dans une direction s'étendant entre lesdites deux régions ; caractérisé par la rotation de la pluralité de régions afin de changer l'orientation desdites au moins deux régions ; la détection du paramètre physique dans ladite direction s'étendant entre les deux régions soumises à une rotation ; et la détermination de la variation directionnelle de la caractéristique physique sur la base du paramètre physique détecté dans les première et seconde directions mentionnées.

12. Procédé selon la revendication 11, dans lequel la feuille est une feuille fibreuse et èn outre comprenant l'étape de déterminer l'angle d'orientation des fibres sur la base de la variation directionnelle de la caractéristique physique.

13. Procédé selon la revendication 11, dans lequel la feuille est une feuille fibreuse et comprenant en outre l'étape de déterminer le degré d'anisotropie des fibres dans la feuille sur la base de la variation directionnelle de la caractéristique physique.

FIG. 1

FIG. 5

FIG. 2

FIG. 3

FIG. 4